# EUROPÄISCHE PATENTANMELDUNG

(11) **EP 1 398 633 A2**
(43) Veröffentlichungstag der Anmeldung: **17.03.2004**
(21) Anmeldenummer: 03019575.4
(22) Anmeldetag: 03.09.2003
(51) Int. Cl.: G01N 33/50, C12Q 1/48

(54) **Verfahren zum Identifizieren von Fungiziden**

(30) Priorität: 16.09.2002 DE 10242940
(71) Anmelder: Bayer CropScience AG, 40789 Monheim (DE)
(72) Erfinder: Vaupel, Martin, Dr., 42799 Leichlingen (DE); Guth, Oliver, Dr., 51371 Leverkusen (DE); Kuck, Karl-Heinz, Dr., 40764 Langenfeld (DE)

(57) **Zusammenfassung**

Die Erfindung bezieht sich auf ein Verfahren zum Identifizieren von Fungiziden, die Verwendung von Famesylpyrophosphat Synthase zum Identifizieren von Fungiziden, und die Verwendung von Inhibitoren der Farnesylpyrophosphat Synthase als Fungizide.

## Beschreibung

Die Erfindung bezieht sich auf ein Verfahren zum Identifizieren von Fungiziden, die Verwendung von Farnesylpyrophosphat Synthase zum Identifizieren von Fungiziden, und die Verwendung von Inhibitoren der Farnesylpyrophosphat Synthase als Fungizide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu identifizieren und zugänglich zu machen, und darauf basierend ein Verfahren zur Verfügung zu stellen, das die Identifizierung von Modulatoren dieses Angriffspunkts ermöglicht, und damit letztlich neue Fungizide zugänglich zu machen.

### Beschreibung der Abbildungen

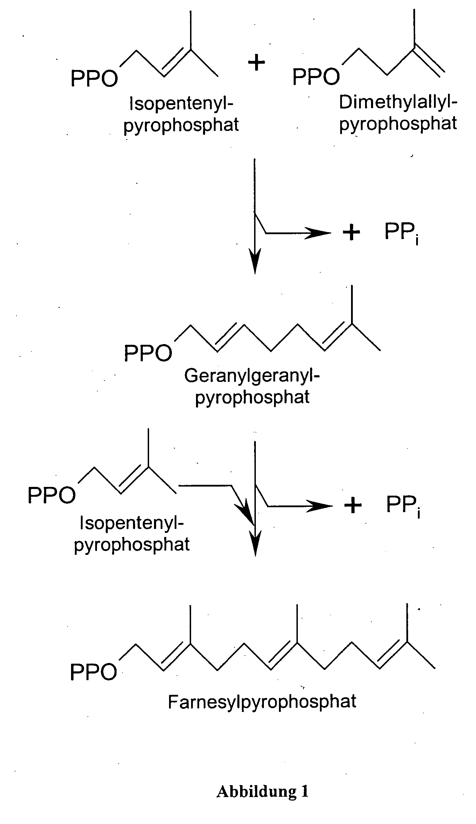

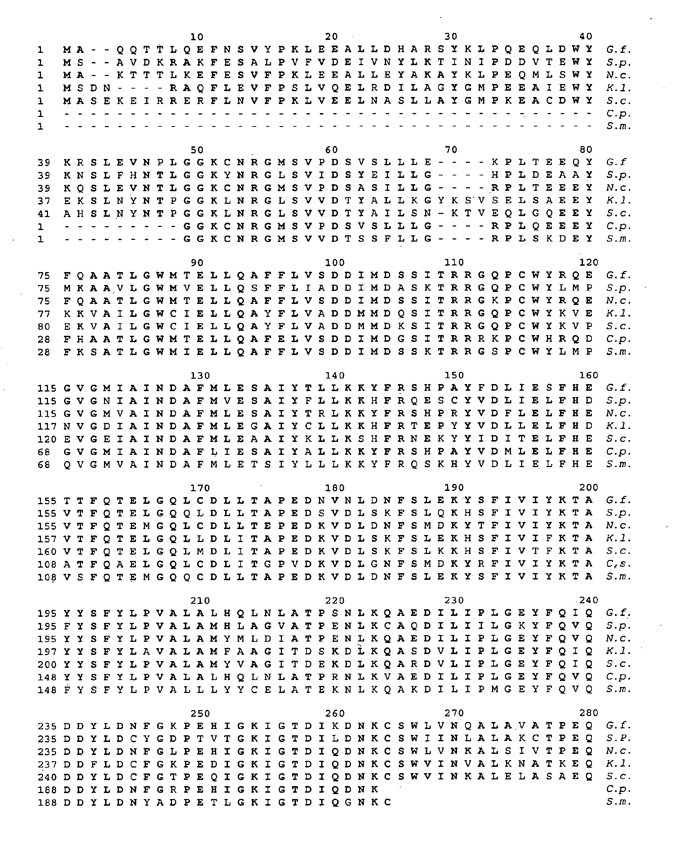

- Abbildung 1:: Die Farnesylpyrophosphat Synthase katalysiert die Reaktion von Dimethylallylpyrophosphat und Isopentenylpyrophosphat zu Pyrophosphat und Geranylpyrophosphat, auf das anschließend ein weiteres Molekül Isopentenylpyrophosphat übertragen wird. Bei der Reaktion werden insgesamt zwei Moleküle Pyrophosphat freigesetzt.

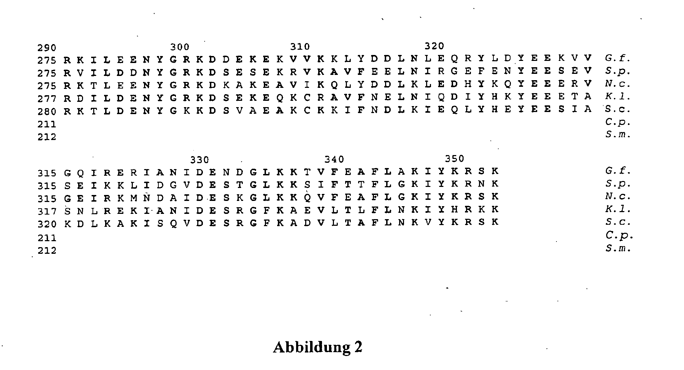

- Abbildung 2:: Homologie zwischen Famesylpyrophosphat Synthasen aus verschiedenen Pilzen: (1) *Saccharomycs cerevisiae (S.c.),* (2) *Neurospora crassa (N.c.),* (3) *Schizosaccharomyces pombe (S.p.),* (4) *Gibberella fujikuroi (G.f),* (5) *Kluyveromyces lactis (K.l.),* (6) *Claviceps purpurea (C.p.),* und (7) *Sphaceloma manihoticola* (S.m.). Rahmen geben Bereiche mit einer genau übereinstimmenden Sequenz wieder (Konsensussequenz).

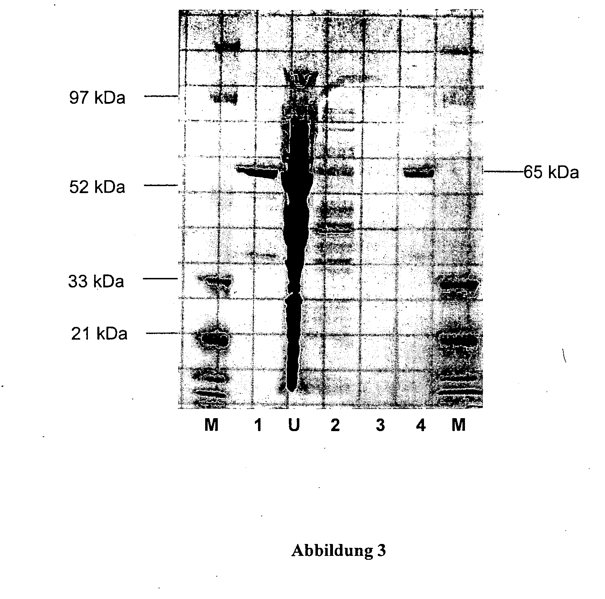

- Abbildung 3:: Heterologe Expression der Farnesylpyrophosphat Synthase in *E*. *coli* Origami. Das überexprimierte GST-Fusionsprotein hat eine Grösse von 65 kDa. In Spur M wurde ein Größenstandard aufgetragen. Spur 1: gereinigte Farnesylpyrophosphat Synthase; Spur 2: Cytoplasmafraktion der überexprimierten Farnesylpyrophosphat Synthase 4 Stunden nach der Induktion mit IPTG; Spur 2 und 3: Waschfraktionen nach dem Auftragen der Cytoplasmafraktion auf die Glutathion-Sepharose Säule; Spur 4: Elutionsfraktion mit gereinigter Farnesylpyrophosphat Synthase.

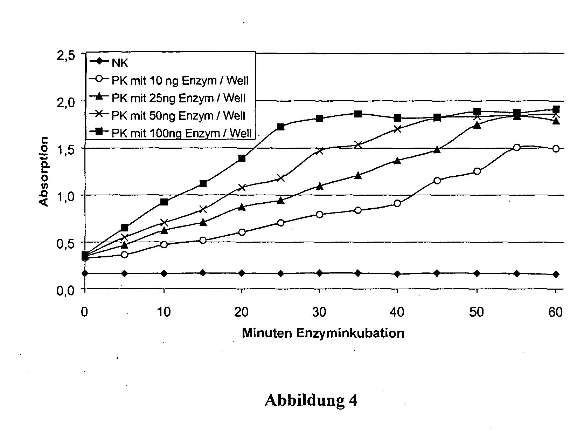

- Abbildung 4:: Kinetik der Umsetzung von Dimethylallylpyrophosphat und Isopentenylpyrophosphat durch unterschiedliche Konzentrationen von Farnesylpyrophosphat Synthase im Assay. In einem Assayvolumen von 40 µl wurden 42 µM Isopentenylpyrophosphat, 54 µM Dimethylallylpyrophosphat, 0,34 mU anorganischer Pyrophosphatase und 0,05 µg Farnesylpyrophosphat Synthase eingesetzt. Die verwendeten Proteinkonzentrationen der Farnesylpyrophosphat Synthase sind der Abbildung zu entnehmen. Die Umsetzung wurde anhand der Absorptionszunahme bei 620 nm durch die Reaktion des freigesetzten Orthophosphates mit Malachitgrün Lösung verfolgt.

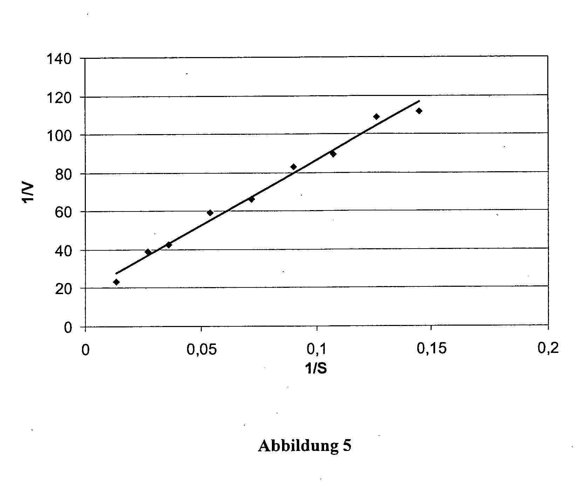

- Abbildung 5:: K_{M}-Wert Bestimmung für Dimethylallylpyrophosphat. Darstellung der gemessenen Werte nach Lineweaver und Burk: 1/Vₒ = 1/Vₘₐₓ + 1/S x (K_{M}/Vₘₐₓ), worin Vₒ die anfängliche Reaktionsgeschwindigkeit, Vₘₐₓ die maximal erreichbare Umsatzgeschwindigkeit und S die Substratkonzentarion ist. Vₘₐₓ und K_{M} lassen sich dann als Abszissen- und Ordinatenabschnitt 1/Vₘₐₓ bzw. 1/K_{M} ablesen.

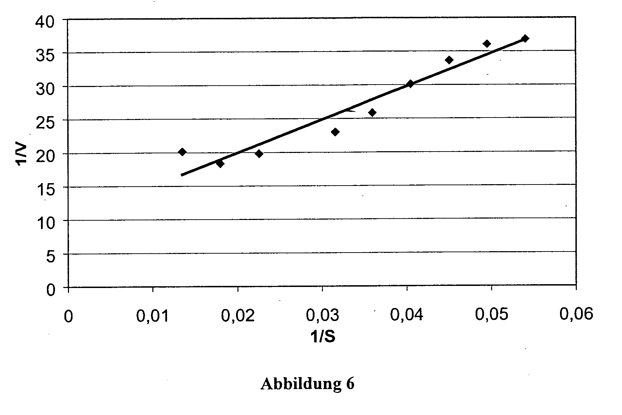

- Abbildung 6:: K_{M}-Wert Bestimmung für Isopentenylpyrophosphat. Darstellung der gemessenen Werte nach Lineweaver und Burk: 1/Vₒ = 1/Vₘₐₓ + 1/S x (K_{M}/Vₘₐₓ), worin Vₒ die anfängliche Reaktionsgeschwindigkeit, Vₘₐₓ die maximal erreichbare Umsatzgeschwindigkeit und S die Substratkonzentration ist. Vₘₐₓ und K_{M} lassen sich dann als Abszissen- und Ordinatenabschnitt 1/Vₘₐₓ bzw. 1/K_{M} ablesen.

Die Farnesylpyrophosphat Synthase (EC 2.5.1.1 und 2.5.1.10), auch bekannt als Farnesylpyrophosphat Synthetase, Farnesyldiphosphat Synthase oder Famesyldiphosphat Synthetase, katalysiert zunächst die Reaktion von Dimethylallylpyrophosphat und Isopentenylpyrophosphat zu Pyrophosphat und Geranylpyrophosphat (Dimethylallyltransferase-Reaktion; EC 2.5.1.1). Anschließend wird ein weiteres Molekül Isopentenylpyrophosphat auf Geranylpyrophosphat übertragen und es entstehen Farnesylpyrophosphat und Pyrophosphat (Geranyltranstransferase-Reaktion; EC 2.5.1.10) (Abbildung 1).

Die von der Farnesylpyrophosphat Synthase katalysierten Reaktionen sind essentielle Schritte für die Bereitstellung von Farnesylpyrophosphat für die Ergosterol-, Dolichol- oder Ubichinon Biosynthese (Lees et al., 1997, Biochemistry and molecular biology of sterol synthesis in *Saccharomyces cerevisiae*. *Biochemistry and Function of Sterols,* 85-99; Mercer, 1984, The biosynthesis of ergosterol. *Pestic. Sci.* 15(2), 133-55; Szkopinska et al., 1997, Polyprenol formation in the yeast *Saccharomyces cerevisiae:* effect of farnesyl diphosphate synthase overexpression. *Journal of Lipid Research* 38(5), 962-968).

Gene für die Farnesylpyrophosphat Synthase wurden aus verschiedenen Pilzen kloniert, aus den Ascomyceten *Saccharomyces cerevisiae* (Swissprot Accession No.: P08524), *Schizosaccharomyces pombe* (Swissprot Accession No.: 014230) und *Neurospora crassa* (Swissprot Accession No.: Q92250) sowie dem pflanzenpathogenen Pilz *Gibberella fujikuroi* (Swissprot Accession No.: Q92235). Sequenzfragmente sind ebenfalls bekannt aus den pflanzenpathogenen Pilzen *Claviceps purpurea* und *Sphaceloma manihoticola.* Daneben wurde die Farnesylpyrophosphat Synthase auch aus zahlreichen anderen Organismen gewonnen, so z.B. aus *Homo sapiens* (Swissprot: Accession No.: P14324), Tomate (Swissprot: Accession No.: 065004) oder Mais (Swissprot: Accession No.: P49353).

Die Sequenzähnlichkeiten sind innerhalb der eukaryontischen Klassen signifikant, während hingegen die Sequenzidentität zu den bakteriellen Enzymen weniger signifikant ist.

Die Farnesylpyrophosphat Synthase wurde z.B. aus Hefe isoliert, exprimiert, gereinigt und auch charakterisiert (Anderson et al., 1989, Farnesyl diphosphate synthetase. Molecular cloning, sequence, and expression of an essential gene from *Saccharomyces cerevisiae. J Biol. Chem.* 264(32), 19176-84; Eberhardt et al., 1975, Prenyltransferase from *Saccharomyces cerevisiae*. Purification to homogeneity and molecular properties. *Journal of Biological Chemistry* 250(3), 863-6; Song et al., 1994, Yeast farnesyl-diphosphate synthase: Site-directed mutagenesis of residues in highly conserved prenyltransferase domains I and II. *Proc. Natl. Acad. Sci. U.S.A.* 91(8), 3044-8).

Der Begriff "Identität", wie er hierin verwendet wird, bezieht sich auf die Zahl von Sequenzpositionen die in einem Alignment identisch sind. Sie wird meist in Prozent der Alignment Länge angegeben.

Der Begriff "Ähnlichkeit", wie er hierin verwendet wird, setzt dagegen die Definition einer Ähnlichkeitsmetrik voraus, also eines Maßes dafür, als wie ähnlich man beispielsweise ein Valin zu einem Threonin oder zu einem Leucin annehmen möchte.

Der Begriff "Homologie", wie er hierin verwendet wird, bedeutet wiederum evolutionäre Verwandtschaft. Zwei homologe Proteine haben sich aus einer gemeinsamen Vorläufersequenz entwickelt. Der Begriff hat nicht unbedingt etwas mit Identität oder Ähnlichkeit zu tun, abgesehen davon, dass homologe Sequenzen meist ähnlicher sind (oder in einem Alignment mehr identische Positionen besitzen) als nichthomologe Sequenzen.

Der Ausdruck "vollständige Farnesylpyrophosphat Synthase" wie er hierin verwendet wird, beschreibt eine Farnesylpyrophosphat Synthase, die von einer vollständigen kodierenden Region einer Transkriptionseinheit kodiert wird, beginnend mit dem ATG-Startcodon und umfassend alle informationstragenden Exonbereiche des im Herkunftsorganismus vorliegenden, für Farnesylpyrophosphat Synthase kodierenden Gens, sowie die für eine korrekte Termination der Transkription nötigen Signale.

Der Ausdruck "biologische Aktivität einer Farnesylpyrophosphat Synthase" wie er hierin verwendet wird, bezieht sich auf die Fähigkeit eines Polypeptids, die vorstehend beschriebene Reaktion, d.h. die Umsetzung von Dimethylallylpyrophosphat und Isopentenylpyrophosphat zu Farnesylpyrophosphat zu katalysieren.

Der Ausdruck "aktives Fragment" wie er hierin verwendet wird, beschreibt nicht mehr vollständige Nukleinsäuren kodierend für Farnesylpyrophosphat Synthase, die aber noch für Polypeptide mit der biologischen Aktivität einer Farnesylpyrophosphat Synthase kodieren, und die eine für die Farnesylpyrophosphat Synthase charakteristische Reaktion wie vorne beschrieben katalysieren können. Solche Fragmente sind kürzer als die oben beschriebenen vollständigen, für die Farnesylpyrophosphat Synthase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der Farnesylpyrophosphat Synthase nicht entscheidend beeinträchtigen. Eine geringere oder gegebenenfalls auch eine erhöhte Aktivität, die aber noch die Charakterisierung bzw. Verwendung des resultierenden Farnesylpyrophosphat Synthase Fragments gestattet, wird dabei als ausreichend im Sinne des hier verwendeten Ausdrucks verstanden. Der Ausdruck "aktives Fragment" kann sich ebenso auf die Aminosäuresequenz der Farnesylpyrophosphat Synthase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Länge besitzen.

Der Begriff "Farnesylpyrophosphat Synthase-Hemmtest" oder "Hemmtest", wie er hierin verwendet wird, bezieht sich auf ein Verfahren bzw. einen Test, der es gestattet, die Inhibition der enzymatischen Aktivität eines Polypeptids mit der Aktivität einer Farnesylpyrophosphat Synthase durch eine oder mehrere chemische Verbindungen (Kandidatenverbindung(en)) zu erkennen, wodurch die chemische Verbindung als Inhibitor der Farnesylpyrophosphat Synthase identifiziert werden kann.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "Fungizid" bzw. "fungizid" wie er hierin verwendet wird, bezieht sich auf chemische Verbindungen, die zur Bekämpfung von Pilzen, insbesondere von pflanzenpathogenen Pilzen geeignet sind. Solche pflanzenpathogene Pilze werden nachfolgenden genannt, wobei die Aufzählung nicht abschließend ist:
Plasmodiophoromycetes, Oomycetes, Chytridiomycetes, Zygomycetes, Ascomycetes, Basidiomycetes und Deuteromycetes, z.B.
Pythium-Arten, wie beispielsweise *Pythium ultimum*, Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P*. *brassicae,* Erysiphe-Arten, wie beispielsweise *Erysiphe graminis*, Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis*, Pyrenophora-Arten, wie beispielsweise *Pyrenophora teres* oder *P*. *graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries*; Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia oryzae,* Fusarium-Arten, wie beispielsweise *Fusarium culmorum*, Botrytis-Arten, Septoria-Arten, wie beispielsweise *Septoria nodorum,* Leptosphaeria-Arten, wie beispielsweise *Leptosphaeria nodorum*, Cercospora-Arten, wie beispielsweise *Cercospora canescens*, Alternaria-Arten, wie beispielsweise *Alternaria brassicae* oder Pseudocercosporella-Arten, wie beispielsweise *Pseudocercosporella herpotrichoides*.
Von besonderem Interesse sind z.B. auch *Magnaporthe grisea, Cochliobulus heterostrophus*, *Nectria hematococcus* und Phytophtora Spezies.

Gegenstand der vorliegenden Erfindung ist deshalb ebenfalls ein Verfahren zur Identifizierung von Fungiziden, d.h. von Inhibitoren der Farnesylpyrophosphat Synthase aus pflanzenpathogenen Pilzen, die als Fungizide zur Bekämpfung von Pilzbefall bei Pflanzen verwendet werden können.

Fungizide Wirkstoffe, die mit Hilfe der erfindungsgemäßen Farnesylpyrophosphat Synthase gefunden werden, können aber auch mit Farnesylpyrophosphat Synthase aus humanpathogenen Pilzspezies interagieren, wobei die Interaktion mit den unterschiedlichen in diesen Pilzen vorkommenden Farnesylpyrophosphat Synthasen nicht immer gleich stark sein muss.

Gegenstand der vorliegenden Erfindungen ist deshalb ein Verfahren zum Identifizieren von Antimykotika, d.h. von Inhibitoren der Farnesylpyrophosphat Synthase aus human- oder tierpathogenen Pilzen zum Herstellen von Mitteln zur Behandlung von durch human- oder tierpathogene Pilze hervorgerufenen Erkrankungen.

Dabei sind die folgenden humanpathogenen Pilze von besonderem Interesse, die unter anderem die nachfolgend genannten Krankheitsbilder hervorrufen können:
Dermatophyten, wie z.B. Trichophyton spec., Microsporum spec., *Epidermophyton floccosum oder Keratomyces ajelloi,* die z.B. Fußmykosen (Tinea pedis) hervorrufen,
Hefen, wie z.B. *Candida albicans,* Soor-Ösophagitis und Dermatitis hervorruft, *Candida glabrata, Candida krusei* oder *Cryptococcus neoformans,* die z.B. pulmonale Cryptococcose und auch Torulose hervorrufen können,
Schimmelpilze, wie z.B. *Aspergillus fumigatus, A*. *flavus, A. niger*, die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi*, der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis*, der z. B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi*, der z.B. Keloid-Blastomykose und Lobo's Krankheit hervorruft, und *Sporothrix schenckii,* der z.B. Sporotrichose (granulomatöse Hautmykose) hervorruft.

Im Folgenden sollen die Begriffe "fungizid" bzw. "Fungizid" gleichermaßen für die Begriffe "antimykotisch" bzw. "Antimykotikum" als auch für die Begriffe "fungizid" bzw. "Fungizid" im herkömmlichen Sinne, d.h. bezogen auf pflanzenpathogene Pilze, verwendet werden.

Fungizide Wirkstoffe, die mit Hilfe einer aus einem bestimmten Pilz, hier z.B. aus *S. cerevisiae*, gewonnenen Farnesylpyrophosphat Synthase gefunden werden, können also auch mit einer Farnesylpyrophosphat Synthase aus zahlreichen anderen Pilzspezies, gerade auch mit pflanzenpathogenen Pilzen interagieren, wobei die Interaktion mit den unterschiedlichen in diesen Pilzen vorkommenden Famesylpyrophosphat Synthasen nicht immer gleich stark sein muss. Dies erklärt unter anderem die beobachtete Selektivität der an diesem Enzym wirksamen Substanzen.

Der Ausdruck "Kompetitor" wie er hierin verwendet wird, bezieht sich auf die Eigenschaft der Verbindungen, mit anderen, gegebenenfalls zu identifizierenden Verbindungen um die Bindung an der Farnesylpyrophosphat Synthase zu kompetitieren und diese vom Enzym zu verdrängen bzw. von dieser verdrängt zu werden.

Der Ausdruck "Agonist", wie er hierin verwendet wird, bezieht sich auf ein Molekül, das die enzymatische Aktivität der Farnesylpyrophosphat Synthase beschleunigt oder verstärkt.

Der Ausdruck "Antagonist", wie er hierin verwendet wird, bezieht sich auf ein Molekül, das die enzymatische Aktivität der Farnesylpyrophosphat Synthase verlangsamt oder zum Erliegen bringt.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

Trotz umfangreicher Forschungen an der Farnesylpyrophosphat Synthase war bislang unbekannt, dass die Farnesylpyrophosphat Synthase in Pilzen ein Zielprotein (ein so genanntes "Target") fungizid wirksamer Substanzen sein kann.

Für bereits bekannte Inhibitoren der Farnesylpyrophosphat Synthase wurde keine fungizide Wirkung beschrieben (siehe z.B. Bergstrom et al., 2000, Alendronate Is a Specific, Nanomolar Inhibitor of Farnesyl Diphosphate Synthase. *Archives of Biochemistry and Biophysics* 373(1), 231-241; Dunford et al., 2001, Structureactivity relationships for inhibition of farnesyl diphosphate synthase in vitro and inhibition of bone resorption in vivo by nitrogen-containing bisphosphonates. *Journal of Pharmacology and Experimental Therapeutics* 296(2), 235-242; Thompson et al., 2002, Identification of a Bisphosphonate That Inhibits Isopentenyl Diphosphate Isomerase and Farnesyl Diphosphate Synthase. *Biochemical and Biophysical Research Communications* 290(2), 869-873).

In der vorliegenden Erfindung wird nun zum ersten Mal gezeigt, dass die Farnesylpyrophosphat Synthase ein insbesondere für Pilze wichtiges Enzym darstellt und deshalb in besonderem Maße dazu geeignet ist, als Zielprotein für die Suche nach weiteren und verbesserten fungizid wirksamen Wirkstoffen verwendet zu werden. Die vorliegende Erfindung zeigt weiterhin, dass das Enzym Farnesylpyrophosphat Synthase weiterhin für Verfahren zum Identifizieren von Modulatoren bzw. Inhibitoren der enzymatischen Aktivität des Polypeptids in geeigneten Testverfahren zugänglich ist, was bei verschiedenen theoretisch interessanten Targets nicht selbstverständlich ist.

Im Rahmen der vorliegenden Erfindung wird weiterhin gezeigt, dass die Farnesylpyrophosphat Synthase auch tatsächlich durch Wirkstoffe beeinflusst werden kann, und dass die Inhibition der pilzlichen Farnesylpyrophosphat Synthase zur Schädigung oder zum Absterben des behandelten Pilzes führt.

So wurde im Rahmen der vorliegenden Erfindung ein Verfahren entwickelt, das geeignet ist, die enzymatische Aktivität der Famesylpyrophosphat Synthase sowie die Hemmung dieser Aktivität durch eine oder mehrere Substanzen in einem so genannten Hemmtest zu bestimmen, und auf diese Weise Inhibitoren des Enzyms z.B. in HTS- und UHTS-Verfahren zu identifizieren. *In vitro* identifizierte Inhibitoren können dann *in vivo* auf ihre fungizide Wirkung geprüft werden.

Im Rahmen der vorliegenden Erfindung wurde weiter gefunden, dass die Farnesylpyrophosphat Synthase auch *in vivo* durch Wirkstoffe inhibiert werden kann und ein mit diesen Wirkstoffen behandelter pilzlicher Organismus durch die Behandlung mit diesen Wirkstoffen geschädigt und abgetötet werden kann. Die Inhibitoren einer pilzlichen Farnesylpyrophosphat Synthase können also als Fungizide insbesondere im Pflanzenschutz oder auch als Antimykotika in Pharmaindikationen verwendet werden. In der vorliegenden Erfindung wird beispielsweise gezeigt, dass die Hemmung der Farnesylpyrophosphat Synthase mit einer der in einem erfindungsgemäßen Verfahren identifizierten Substanzen zu einer Wachstumshemmung oder zum Absterben der behandelten Pilze in synthetischen Medien bzw. auf der Pflanze führt.

Eine Farnesylpyrophosphat Synthase, die in einem erfindungsgemäßen Verfahren eingesetzt werden kann, kann z.B. aus Pilzen wie *S. cerevisiae* gewonnen werden. Zur Herstellung der Farnesylpyrophosphat Synthase aus Hefe kann das Gen z.B. rekombinant in *Escherichia coli* exprimiert und aus *E*. *coli* Zellen eine Enzympräparation hergestellt werden (Beispiel 1).

So wurde für die Expression des von *erg20* kodierten Polypeptids Erg20 (Chambon et al., 1990, Isolation and properties of yeast mutants affected in famesyl diphosphate synthetase. *Curr. Genet.* 18(1), 41-6; SWISS-PROT Accession Nummer: P08524) der zugehörige ORF aus genomischer DNA nach dem Fachmann bekannten Methoden über genspezifische Primer amplifiziert. Die entsprechende DNA wurde in den Vektor pGEX-4T-1 (Pharmacia Biotech, ermöglicht die Einführung eines N-terminalen GST-Tags) kloniert. Das resultierende Plasmid pErg20 enthält die vollständige kodierende Sequenz von erg20 in einer N-terminalen Fusion mit einem GST-Tag aus dem Vektor. Das Erg20 Fusionsprotein besitzt eine berechnete Masse von 64,5 kDa (vgl. Beispiel 1 und Abbildung 3).

Das Plasmid pErg20 wurde dann zur rekombinanten Expression von Erg20 in *E*. *coli* Origami Zellen verwendet (vgl. Beispiel 1).

Wie bereits vorstehend ausgeführt, ist die vorliegende Erfindung nicht nur auf die Verwendung von Famesylpyrophosphat Synthase aus Hefe beschränkt. In analoger und dem Fachmann bekannter Weise können auch aus anderen Pilzen, vorzugsweise aus pflanzenpathogenen Pilzen, Polypeptide mit der Aktivität einer Famesylpyrophosphat Synthase gewonnen werden, die dann in einem erfindungsgemäßen Verfahren eingesetzt werden können. Bevorzugt wird die Farnesylpyrophosphat Synthase aus *S*. *cerevisiae* verwendet.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können. In den erfindungsgemäßen Verfahren können ebenso aktive Fragmente einer Farnesylpyrophosphat Synthase eingesetzt werden, solange sie die Bestimmung der enzymatischen Aktivität des Polypeptids bzw. deren Inhibition durch eine Kandidatenverbindung ermöglichen.

Die in den erfindungsgemäßen Verfahren eingesetzten Polypeptide können im Vergleich zu den entsprechenden Regionen von natürlich vorkommenden Famesylpyrophosphat Synthasen Deletionen oder Aminosäuresubstitutionen aufweisen, solange sie zumindest noch die biologische Aktivität einer vollständigen Farnesylpyrophosphat Synthase zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Ein mögliches Reinigungsverfahren der Farnesylpyrophosphat Synthase basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie (vgl. Beispiel 1).

Ein schnelles Verfahren zum Isolieren von Farnesylpyrophosphat Synthasen, die von Wirtszellen synthetisiert werden, beginnt mit der Expression eines Fusionsproteins, wobei der Fusionspartner auf einfache Weise affinitätsgereinigt werden kann. Der Fusionspartner kann beispielsweise ein GST-Tag sein (vgl. Beispiel 1). Das Fusionsprotein kann dann an Glutathion-Sepharosesäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Reversphasenoder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Das Verfahren zum Herstellen von Polypeptiden mit der Aktivität einer Farnesylpyrophosphat Synthase, wie z.B. des Polypeptids Erg20, ist damit gekennzeichnet ist durch
(a) das Kultivieren einer Wirtszelle enthaltend zumindest eine exprimierbare Nukleinsäuresequenz kodierend für ein Polypeptid aus Pilzen mit der biologischen Aktivität einer Farnesylpyrophosphat Synthase unter Bedingungen, die die Expression dieser Nukleinsäure gewährleisten, oder
(b) das Exprimieren einer exprimierbaren Nukleinsäuresequenz kodierend für ein Polypeptid aus Pilzen mit der biologischen Aktivität einer Famesylpyrophosphat Synthase in einem *in vitro*-System, und
(c) die Gewinnung des Polypeptids aus der Zelle, dem Kulturmedium oder dem *in vitro*-System.
Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der Farnesylpyrophosphat Synthase verwendet zu werden.

Gegenstand der vorliegenden Erfindung ist insbesondere auch die Verwendung von Polypeptiden aus Pilzen, welche zumindest eine biologische Aktivität einer Famesylpyrophosphat Synthase ausüben in Verfahren zum Identifizieren von Inhibitoren eines Polypeptids aus Pilzen mit der Aktivität einer Farnesylpyrophosphat Synthase, wobei die Inhibitoren der Farnesylpyrophosphat Synthase als Fungizide verwendet werden können. Besonders bevorzugt wird die Farnesylpyrophosphat Synthase aus *S*. *cerevisiae* verwendet.

Fungizide Wirkstoffe, die mit Hilfe einer Farnesylpyrophosphat Synthase aus einer bestimmten Pilzspezies gefunden werden, können auch mit Farnesylpyrophosphat Synthasen aus anderen Pilzspezies interagieren, wobei die Interaktion mit den unterschiedlichen in diesen Pilzen vorkommenden Farnesylpyrophosphat Synthase nicht immer gleich stark sein muss. Dies erklärt unter anderem die Selektivität wirksamer Substanzen. Die Verwendung von Wirkstoffen, die mit einer Famesylpyrophosphat Synthase einer bestimmten Pilz-Spezies gefunden wurden, als Fungizide auch bei anderen Pilze-Spezies kann darauf zurückgeführt werden, dass sich Farnesylpyrophosphat Synthasen aus verschiedenen Pilzspezies sehr nahe stehen und in größeren Bereichen eine ausgeprägte Homologie zeigen. So wird in Abbildung 2 deutlich, dass eine solche Homologie über beträchtliche Sequenzabschnitte hinweg zwischen *S. cerevisiae, N. crassa, S. pombe, K. lactis, S. manihoticola, C. purpurea* und *G. fujikuroi* besteht und damit die Wirkung der mit Hilfe der Farnesylpyrophosphat Synthase aus Hefe gefundenen Substanzen nicht auf *S. cerevisiae* beschränkt bleibt. In Verfahren zum Identifizieren von Fungiziden werden deshalb bevorzugt Polypeptide mit der enzymatischen Aktivität einer Farnesylpyrophosphat Synthase verwendet, die eine Konsensussequenz gemäß Abbildung 2 aufweisen.

Gegenstand der vorliegenden Erfindung ist weiterhin ein Verfahren zum Identifizieren von Fungiziden durch Testen von potentiellen Inhibitoren bzw. Modulatoren der enzymatischen Aktivität der Farnesylpyrophosphat Synthase (Kandidatenverbindung) in einem Farnesylpyrophosphat Synthase-Hemmtest.

Verfahren, die geeignet sind, Modulatoren, insbesondere Inhibitoren bzw. Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren (*in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden. Im Anschluss an die *in vivo* oder *in vitro* Identifizierung von Modulatoren des Polypeptids können Tests an Pilzkulturen durchgeführt werden, um die fungizide Wirksamkeit der gefundenen Verbindungen zu prüfen.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semigereinigtes Protein. Sie sind geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit eines Kandidatenmoleküls, das ein Antagonist sein kann, inkubiert werden. Die Fähigkeit des Kandidatenmoleküls, die Aktivität der erfindungsgemäßen Polypeptide zu hemmen, wird z.B. erkennbar an einer verringerten Bindung des gegebenenfalls markierten Liganden oder an einer verringerten Umsetzung des gegebenenfalls markierten Substrates. Moleküle, die die biologische Aktivität der erfindungsgemäßen Polypeptide hemmen, sind gute Antagonisten.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrische nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Durch die anhand der vorliegenden Erfindung verfügbaren, für eine erfindungsgemäße Farnesylpyrophosphat Synthase kodierende Nukleinsäuren enthaltenden Wirtszellen, wird die Entwicklung von Testsystemen, die auf Zellen basieren, zur Identifizierung von Substanzen ermöglicht, die die Aktivität der erfindungsgemäßen Polypeptide modulieren.

Eine andere Möglichkeit zur Identifizierung von Substanzen, die die Aktivtät der erfindungsgemäßen Polypeptide modulieren, ist so auch der sogenannte "Scintillation Proximity Assay" (SPA), siehe EP 015 473. Dieses Testsystem nutzt die Interaktion eines Polypeptids (z.B. Farnesylpyrophosphat Synthase aus Hefe) mit einem radiomarkierten Liganden bzw. Substrat. Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

Vorzugsweise handelt es sich bei den zu identifizierenden Modulatoren um kleine organisch-chemische Verbindungen.

Ein Verfahren zum Identifizieren einer Verbindung, die die Aktivität einer Farnesylpyrophosphat Synthase aus Pilzen moduliert und die als Fungizid im Pflanzenschutz verwendet werden kann, besteht bevorzugt darin, dass man
a) ein erfindungsgemäßes Polypeptid oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
b) die Aktivität des erfindungsgemäßen Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
c) die chemische Verbindung bestimmt, die die Aktivität des erfindungsgemäßen Polypeptids spezifisch moduliert und gegebenenfalls
d) die fungizide Wirkung der bestimmten Verbindung *in vivo* prüft.

Besonders bevorzugt wird dabei diejenige Verbindung bestimmt, die die Aktivität des erfindüngsgemäßen Polypeptids spezifisch inhibiert. Der Begriff "Aktivität", wie er hier verwendet wird, bezieht sich auf die biologische Aktivität des erfindungsgemäßen Polypeptids.

Ein bevorzugtes Verfahren nutzt die Tatsache aus, dass bei der Reaktion der Farnesylpyrophosphat Synthase zwei Moleküle Pyrophosphat freigesetzt werden. Die Aktivität bzw. die Ab- oder Zunahme der Aktivität des erfindungsgemäßen Polypeptids kann deswegen durch enzymatische Spaltung des Pyrophosphats mittels anorganischer Pyrophosphatase und anschließendem Nachweis des freigesetzten Orthophosphats mit einem Phosphatnachweisreagenz bestimmt werden. Dabei wird die geringere bzw. inhibierte Aktivität des erfindungsgemäßen Polypeptids anhand der photospektrometrischen Bestimmung der Abnahme bzw. der Zunahme des freigesetzten Orthophosphats verfolgt. Die Konzentration des freigesetzten Phosphat kann dann mit einem Phosphatnachweisreagenz bei einem Absorptionsmaximum bei 620 nm bestimmt werden.

Die Messung kann auch in für HTS- oder UHTS-Assays gängigeren Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in der gewünschten Endkonzentrationen vorliegen (vgl. Beispiel 2). Dabei wird die zu testende, potentiell die Aktivität des Enzyms inhibierende oder aktivierende Verbindung (Kandidatenmolekül) z.B. in einer geeigneten Konzentration in Testpuffer enthaltend Dimethylallylpyrophosphat und Isopentenylpyrophosphat vorgelegt. Dann wird das erfindungsgemäße Polypeptid in Testpuffer und das für den gekoppelten Assay notwendige Hilfsenzym anorganische Pyrophosphatase zugegeben und die Reaktion damit gestartet. Der Ansatz wird dann z.B. bis zu 40 Minuten bei einer geeigneten Temperatur inkubiert und die Absorptionszunahme bei 620 nm gemessen.

Eine weitere Messung erfolgt in einem entsprechenden Ansatz, jedoch ohne Zugabe eines Kandidatenmoleküls und ohne Zugabe eines erfindungsgemäßen Polypeptids (Negativkontrolle). Eine weitere Messung erfolgt wiederum bei Abwesenheit eines Kandidatenmoleküls, jedoch bei Anwesenheit des erfindungsgemäßen Polypeptids (Positivkontrolle). Negativ- und Positivkontrolle ergeben damit die Vergleichswerte zu den Ansätzen bei Anwesenheit eines Kandidatenmoleküls.

Um optimale Bedingungen für ein Verfahren zum Identifizieren von Inhibitoren der Farnesylpyrophosphat Synthase bzw. zur Bestimmung der Aktivität der erfindungsgemäßen Polypeptide zu ermitteln, kann es vorteilhaft sein, den jeweiligen K_{M}-Wert des verwendeten erfindungsgemäßen Polypeptids zu bestimmen. Dieser gibt Aufschluss über die bevorzugt zu verwendende Konzentration des bzw. der Substrate. Im Fall der Farnesylpyrophosphat Synthase aus Hefe konnte ein K_{M} von 36 µM für Dimethylallylpyrophosphat und ein K_{M} von 49 µM für Isopentenylpyrophosphat bestimmt werden (Abbildung 5 und 6).

Mit Hilfe der vorstehend beispielhaft beschriebenen Verfahren konnten im Rahmen der vorliegenden Erfindung Verbindungen identifiziert werden, die die pilzliche Farnesylpyrophosphat Synthase inhibieren, und die in der Lage sind, Pilze verschiedener Spezies zu schädigen (z.B. Wachstumshemmung) bzw. abzutöten.

Es versteht sich von selbst, dass neben den genannten Verfahren zur Bestimmung der enzymatischen Aktivität einer Famesylpyrophosphat Synthase bzw. der Inhibition dieser Aktivität und zum Identifizieren von Fungiziden auch andere, z.B. bereits bekannte, Verfahren bzw. Hemmtests verwendet werden können, solange diese Verfahren es gestatten, die Aktivität einer Farnesylpyrophosphat Synthase zu bestimmen und eine Inhibition dieser Aktivität durch eine Kandidtatenverbindung zu erkennen.

In Tabelle I werden beispielhaft Verbindungen gezeigt, die mit einem erfindungsgemäßen Verfahren als Inhibitoren der Farnesylpyrophosphat Synthase identifiziert werden konnten.

Der dort angegebene pI50-Wert ist der negative dekadische Logarithmus des so genannten IC50-Werts, der die molare Konzentration einer Substanz angibt, die zur 50 %igen Hemmung des Enzyms führt.

Ein pI50-Wert von 8 entspricht z.B. einer halbmaximalen Hemmung des Enzyms bei einer Konzentration von 10 nM.

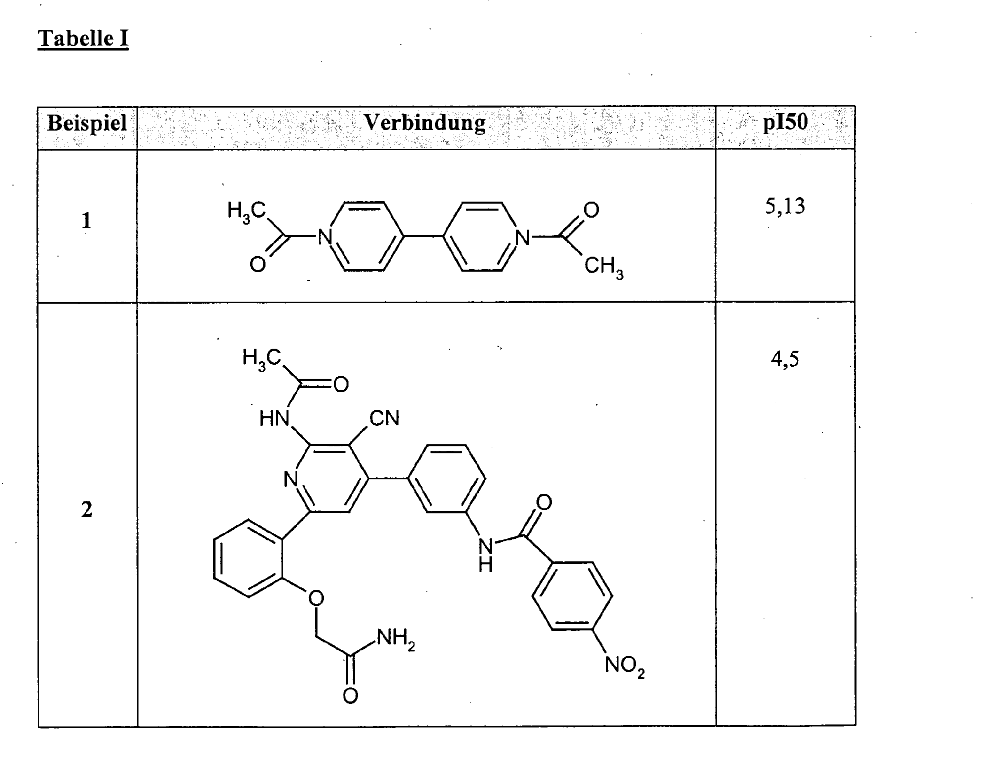

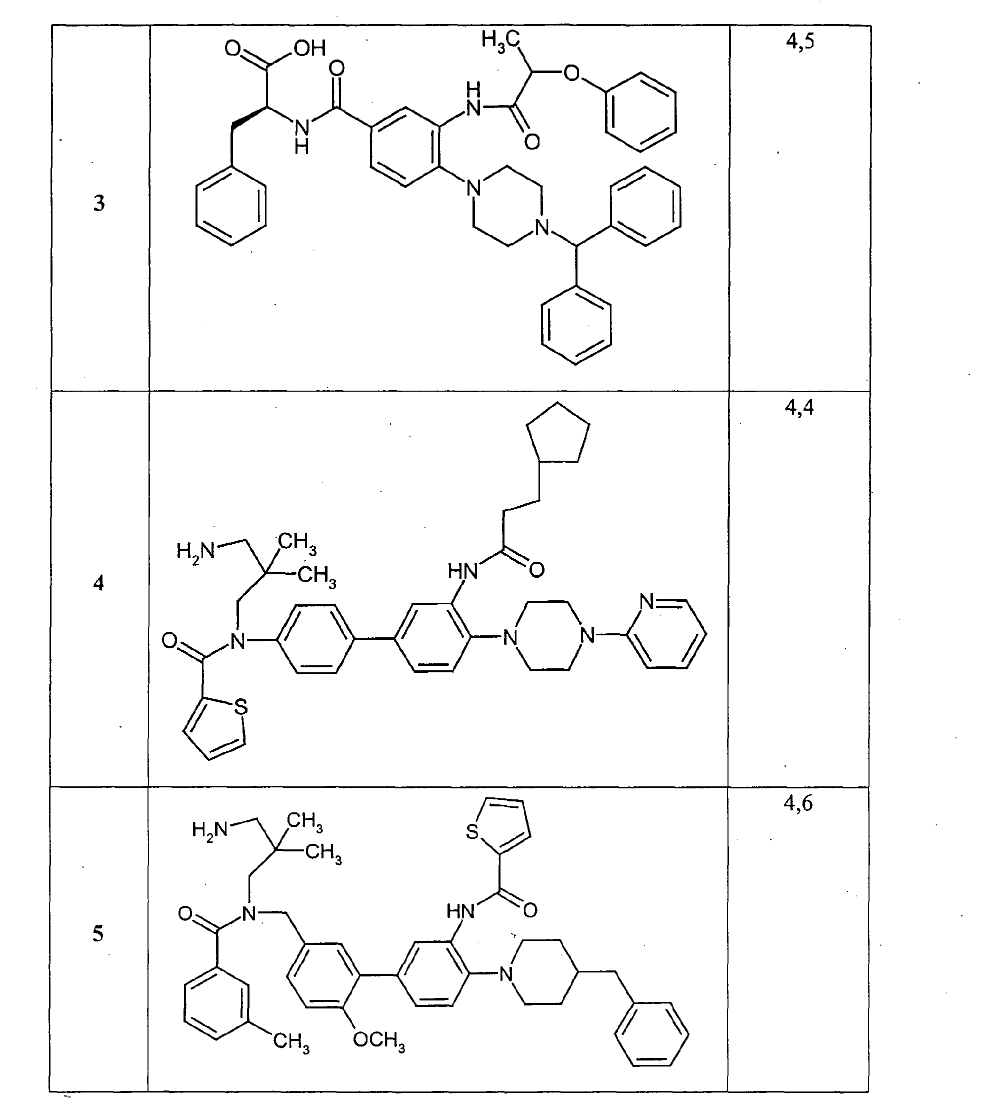

Im Rahmen der vorliegenden Erfindung konnte weiter gezeigt werden, dass die mit Hilfe eines erfindungsgemäßen Verfahrens identifizierten Inhibitoren einer erfindungsgemäßen Farnesylpyrophosphat Synthase geeignet sind, Pilze zu schädigen oder zu töten.

Dazu kann z.B. in die Kavitäten von Mikrotiterplatten eine Lösung des zu prüfenden Wirkstoffs pipettiert werden. Nachdem das Lösungsmittel abgedampft ist, wird zu jeder Kavität Medium hinzugefügt. Das Medium wird vorher mit einer geeigneten Konzentration von Sporen bzw. Mycel des zu prüfenden Pilzes versetzt. Die resultierenden Konzentrationen des Wirkstoffes betragen z.B. 0,1, 1, 10 und 100 ppm.

Die Platten werden anschließend auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen kann die Wirkstoffdosis bestimmt werden, die zu einer 50 %igen Hemmung des Pilzwachstums gegenüber der unbehandelten Kontrolle führt (ED₅₀). In Tabelle II sind die Ergebnisse eines solchen Tests als ED₅₀-Werte für in einem erfindungsgemäßen Verfahren gefundene Verbindungen (Tab. I) beispielhaft wiedergegeben.

Die Wirkung der mit Hilfe eines erfindungsgemäßen Verfahrens gefundenen Verbindungen auf Pilze kann auch getestet werden, indem man ihre protektive Wirkung für Pflanzen prüft. Dazu wird eine zweckmäßige Wirkstoffzubereitung hergestellt. Man vermischt z.B. 1 Gewichtsteil Wirkstoff mit z.B. 24,5 Gewichtsteilen Aceton und 24,5 Gewichtsteilen Dimethylformamid und 1 Gewichtsteil Alkyl-Aryl-Polyglykolether als Emulgator und verdünnt das Konzentrat auf die gewünschte Konzentration.

Zur Prüfung der protektiven Wirkung werden junge Pflanzen mit der Wirkstoffzubereitung besprüht. Nach Antrocknung des Spritzbelages werden die Pflanzen mit einer wässrigen Suspension (Konidiensuspension) eines Pilzes inokuliert und verbleiben dann 1 Tag bei ca. 20°C und 100 % relativer Luftfeuchtigkeit in einer Inkubationskabine.

Die Pflanzen werden dann im Gewächshaus bei ca. 12°C und einer relativen Luftfeuchtigkeit von ca. 90 % aufgestellt.

1 bis 12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In Tabelle II wird für verschiedene Verbindungen gemäß Tabelle I angegeben, bei welcher Konzentration in diesem Test ein Wirkungsgrad von 50 % erreicht wurde. Die betreffenden Beispiele sind an der Angabe einer Trägerpflanze zu erkennen.

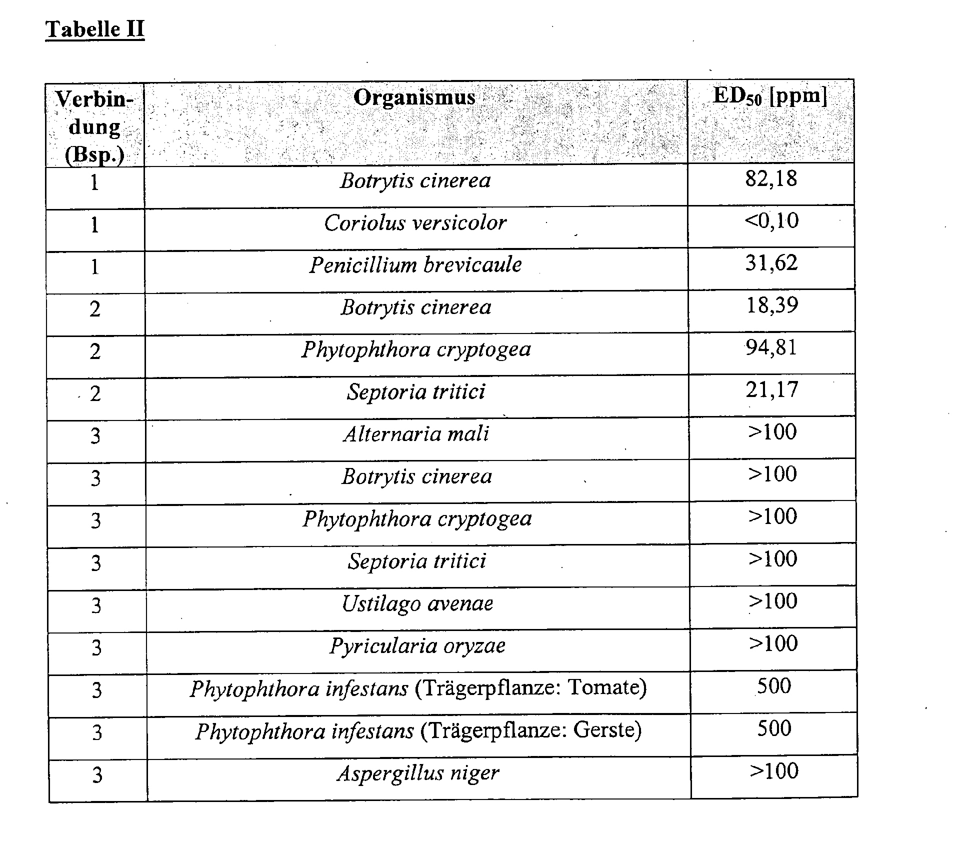

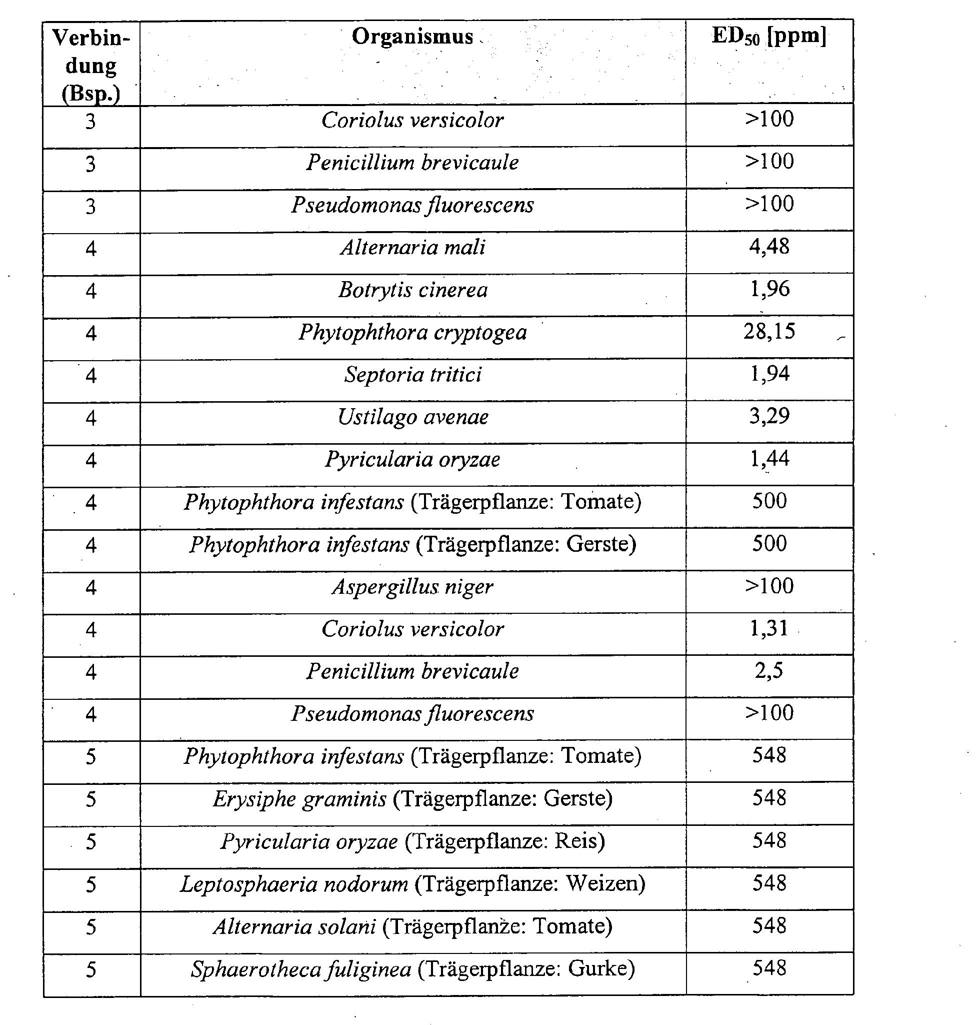

Die vorliegende Erfindung bezieht sich daher ebenfalls auf die Verwendung von Modulatoren der Farnesylpyrophosphat Synthase aus Pilzen, bevorzugt aus pflanzenpathogenen Pilzen als Fungizide.

Die vorliegende Erfindung bezieht sich auch auf Fungizide, die mit Hilfe eines erfindungsgemäßen Verfahrens identifiziert wurden.

Verbindungen, die mit Hilfe eines erfindungsgemäßen Verfahrens identifiziert werden, und die aufgrund der Inhibition der pilzlichen Farnesylpyrophosphat Synthase eine fungizide Wirkung aufweisen, können dann zur Herstellung von fungiziden Mitteln verwendet werden.

Die identifzierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die nachfolgenden Beispiele illustrieren verschiedene Aspekte der vorliegenden Erfindung und sind nicht limitieren auszulegen.

### Beispiele

### Beispiel-1

### Klonierung, Expression und Reinigung von erg20 bzw. Erg20 aus Saccharomyces cerevisiae

Für die Klonierung von erg20 bzw. dessen Expression wurde der ORF aus genomischer DNA aus *Saccharomyces cerevisiae* über genspezifische Primer amplifiziert. Die entsprechende DNA, ein Amplikon von 1059 bp Länge, wurde in den Vektor pGEX-4T-1 von Pharmacia Biotech zwischenkloniert und anschließend über die durch die Primer eingeführten Schnittstellen *Bam*HI und *Xho*I in den mit Schnittstellen *Bam*HI und *Xho*I geschnittenen Vektor pGEX-4T-1 (Pharmacia Biotech) kloniert. Das resultierende Plasmid pErg20 enthält die vollständige kodierende Sequenz von *erg20* in einer N-terminalen Fusion mit dem GST-Tag, das Bestandteil des Vektors ist. Das Erg20 Fusionsprotein besitzt eine berechnete Masse von 64,5 kDa.

Für die heterologe Expression wurde das Plasmid pErg20 so in *E. coli* Origami transformiert, dass der Transformationsansatz direkt als Vorkultur in 50 ml Selektionsmedium diente. Diese Zellen wurden über Nacht bei 37°C inkubiert und anschließend 1:25 in Selektionsmedium (LB-Medium mit 100 µg/ml Ampicillin) verdünnt. Die Induktion erfolgte bei einer OD₆₀₀ₙₘ von 0,8 - 1,0 mit 0,5 mM IPTG (Endkonzentration) bei 37°C. Nach 4 h Induktion wurden die Zellen geerntet und bei -20°C gelagert. Der Aufschluss erfolgte durch Sonifizieren in Lysepuffer (50 mM Tris-HCl, pH 7, 1 mM DTT, 1 mM EDTA, 10 % Glycerin). Die durch Zentrifugation (20 min bei 4°C, 10 000 g) erhaltene Cytoplasmafraktion wurde für die Aufreinigung des exprimierten Proteins verwendet. Die Reinigung erfolgte nach dem Standartprotokoll des Herstellers für Glutathion-Sepharose Säulen unter Verwendung eines Sorbitolspuffers (100 mM Tris/HCl, pH 7,3; 300 mM Sorbitol, 100 mM NaCl, 5 mM MgCl₂). Als Elutionspuffer wurde 50 mM Tris/HCl pH 8,0 mit 10 mM reduziertem Glutathion verwendet. Das gereinigte Protein wurde in Puffer mit Glycerin versetzt (50 mM Tris-HCl pH 8,0, 10 mM Glutathion, 10 % Glycerin) und bei -80°C gelagert. Aus 250 ml Kulturmedium konnte etwa 2,0 mg lösliches Protein isoliert werden, das in Verfahren zum Identifizieren von Modulatoren der Farnesylpyrophosphat Synthase verwendet werden konnte.

### Beispiel 2

### Identifzierung von Modulatoren der Farnesylpyrophosphat Synthase in 384-Well-MTP in einem gekoppelten Assay

Zur Identifizierung von Modulatoren der Farnesylpyrophosphat Synthase wurden 384-Well-Mikrotiterplatten von Greiner verwendet.

In die erste Spalte wurde die Negativ-Kontrolle pipettiert. Diese setzte sich zusammen aus 5 µl Testpuffer (50 mM Tris/HCl pH 7,5, 3 mM MgCl₂, 2 mM DTT, 0,01 % Tween 20) mit 5 % DMSO, 20 µl Mix1 (50 mM Tris/HCl pH 7,5, 3 mM MgCl₂, 2 mM DTT, 0,01 % Tween 20, 42 µM Isopentenylpyrophosphat, 54 µM Dimethylallylpyrophosphat) und 20 µl Testpuffer (50 mM Tris/HCl pH 7,5, 3 mM MgCl₂, 2 mM DTT, 0,01 % Tween 20) mit 0,34 mU anorganischer Pyrophosphatase.

In die zweite Spalte wurde die Positiv-Kontrolle pipettiert. Diese setzte sich zusammen aus 5 µl Testpuffer mit 5% DMSO, 20 µl Mix 1 (50 mM Tris/HCl pH 7,5, 3 mM MgCl₂, 2 mM DTT, 0,01 % Tween 20, 42 µM Isopentenylpyrophosphat, 54 µM Dimethylallylpyrophosphat) und 20 µl Mix 2 (50 mM Tris/HCl pH 7,5, 3 mM MgCl₂, 2 mM DTT, 0,01 % Tween 20, 0,34 mU anorganischer Pyrophosphatase, 0,05 µg Farnesylpyrophosphat Synthase).

In die verbleibenden Spalten wurde eine Prüfsubstanz in einer Konzentration von 2 µM in DMSO vorgelegt, wobei zum Verdünnen der Substanz auf ein Volumen von 5 µl der Testpuffer verwendet wurde. Nach Zugabe von 20 µl Mix 1 (50 mM Tris/HCl pH 7,5, 3 mM MgCl₂, 2 mM DTT, 0,01 % Tween 20, 42 µM Isopentenylpyrophosphat, 54 µM Dimethylallylpyrophosphat) wurden zum Start der Reaktion 20 µl Mix 2 (50 mM Tris/HCl pH 7,5, 3 mM MgCl₂, 2 mM DTT, 0,01 % Tween 20, 0,34 mU anorganischer Pyrophosphatase, 0,05 µg Farnesylpyrophosphat Synthase) zugegeben. Es folgte Inkubation bei Raumtemperatur für 40 Minuten. Anschließend wird die Reaktion durch Zugabe von 50 µl Malachitgrünlösung (drei Teile 0,025 % Malachitgrün Lösung (in Wasser) wurden mit einem Teil 2 % Ammoniumheptamolybdat Lösung (in 4 M HCl) gemischt und direkt vor der Testung 39 Teile dieser Lösung mit einem Teil 7,5 % Tween 20 (in Wasser) gemischt) abgestoppt und 90 Minuten bei Raumtemperatur inkubiert. Die Messung des während der Reaktion entstehenden Orthophosphats erfolgte durch Bestimmung der Absorption bei 620 nm in einem für MTP geeigneten SPECTRAFluor Plus von Tecan.

### Beispiel 3

### Nachweis der fungiziden Wirkung der identifizierten Inhibitoren der Farnesylpyrophosphat Synthase

In die Kavitäten von Mikrotiterplatten wurde eine methanolische Lösung des anhand eines erfindungsgemäßen Verfahrens identifizierten Wirkstoffs (Tab. I) in der gewünschten Menge, versetzt mit einem Emulgator, pipettiert. Nachdem das Lösungsmittel abgedampft war, wurden je Kavität 200 µl Potatoe-Dextrose-Medium hinzugefügt. Das Medium wurde vorher mit geeigneten Konzentrationen von Sporen bzw.
Mycelen des zu prüfenden Pilzes (siehe Tabelle II) versetzt.

Die resultierende Konzentration des Emulgators betrug 300 ppm.

Die Platten wurden anschließend auf einem Schüttler bei einer Temperatur von 22°C inkubiert, bis in der unbehandelten Kontrolle ein ausreichendes Wachstum feststellbar war. Die Auswertung erfolgte photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet (siehe Tab. II).

## Patentansprüche

1. Verfahren zum Identifizieren von Fungiziden, **dadurch gekennzeichnet, dass** man eine chemische Verbindung in einem Farnesylpyrophosphat Synthase-Hemmtest testet.

2. Verfahren gemäß Anspruch 1, **dadurch gekennzeichnet, dass** man
(a) eine Wirtszelle, die eine Farnesylpyrophosphat Synthase in ausreichender Menge exprimiert oder ein Polypeptid mit der enzymatischen Aktivität einer Farnesylpyrophosphat Synthase mit einer chemischen Verbindung oder einem Gemisch von chemischen Verbindungen unter Bedingungen, die die Interaktion der chemischen Verbindung mit dem Polypeptid erlauben, in Kontakt bringt,
(b) die Aktivität der Farnesylpyrophosphat Synthase bei Abwesenheit einer chemischen Verbindung mit der Aktivität der Farnesylpyrophosphat Synthase bei Anwesenheit einer chemischen Verbindung oder eines Gemisches von chemischen Verbindungen vergleicht, und
(c) die chemische Verbindung, die die Farnesylpyrophosphat Synthase spezifisch inhibiert, bestimmt.

3. Verfahren gemäß Anspruch 1 oder 2, **dadurch gekennzeichnet, dass** man eine Farnesylpyrophosphat Synthase aus einem Pilz verwendet.

4. Verfahren gemäß einem der Ansprüche 1 bis 3, **dadurch gekennzeichnet, dass** die Hemmung der enzymatischen Aktivität der Farnesylpyrophosphat Synthase anhand der mit einem Phosphatnachweisreagenz bestimmten Menge der abgespalteten Phosphatgruppe von D-Mannose-1-phosphat gemessen wird.

5. Verfahren gemäß einem der Ansprüche 1 bis 4, **dadurch gekennzeichnet, dass** man in einem weiteren Schritt die fungizide Wirkung der identifizierten Verbindung testet, indem man sie mit einem Pilz in Kontakt bringt.

6. Verwendung von Polypeptiden mit der Aktivität einer Farnesylpyrophosphat Synthase zum Identifizieren von Fungiziden.

7. Verwendung von Inhibitoren von Polypeptiden mit der Aktivität einer Farnesylpyrophosphat Synthase als Fungizide.

8. Verwendung von Inhibitoren eines Polypeptids mit der Aktivität einer Farnesylpyrophosphat Synthase, welche durch ein Verfahren gemäß einem der Ansprüche 1 bis 4 identifiziert werden, als Fungizide.

9. Verwendung von fungiziden Verbindungen, die in einem Verfahren gemäß einem der Ansprüche 1 bis 5 gefunden werden, zum Herstellen von fungiziden Mitteln.
